# EUROPÄISCHE PATENTSCHRIFT

(11) **EP 3 409 648 B1**
(45) Veröffentlichungstag und Bekanntmachung des Hinweises auf die Patenterteilung: **06.01.2021**
(21) Anmeldenummer: 17174068.1
(22) Anmeldetag: 01.06.2017
(51) Int. Cl.: C03C 3/097, C03C 4/00, C03C 10/00

(54) **LITHIUMSILIKAT-GLASKERAMIK MIT SCHEELIT- ODER POWELLIT-KRISTALLPHASE**
LITHIUM SILICATE GLASS CERAMIC WITH SCHEELITE OR POWELLITE CRYSTAL PHASE
VITROCÉRAMIQUE À BASE DE SILICATE DE LITHIUM AYANT UNE PHASE CRISTALLINE POWELLITE OU SCHEELITE

(43) Veröffentlichungstag der Anmeldung: 05.12.2018
(73) Patentinhaber: Ivoclar Vivadent AG, 9494 Schaan (LI)
(72) Erfinder: Dittmer, Marc, 6800 Feldkirch (AT); Ritzberger, Christian, 9472 Grabs (CH); Rampf, Markus, 8853 Lachen (CH)
(74) Vertreter: Uexküll & Stolberg

(56) Entgegenhaltungen:
- EP-A1- 3 150 563
- WO-A1-2015/173394

## Beschreibung

Die Erfindung betrifft Lithiumsilikat-Glaskeramik mit Scheelit und/oder Powellit als weiterer Kristallphase, welche sich insbesondere zur Verwendung als Dentalmaterial, bevorzugt zur Herstellung von Dentalrestaurationen, eignet sowie Vorstufen zur Herstellung dieser Glaskeramik.

Lithiumsilikat-Glaskeramiken zeichnen sich durch sehr gute mechanische Eigenschaften aus, weshalb sie seit langem im Dentalbereich und dort vornehmlich zur Herstellung von Dentalkronen und kleinen Brücken Anwendung finden. Die bekannten Lithiumsilikat-Glaskeramiken enthalten üblicherweise als Hauptkomponenten SiO₂, Li₂O, Al₂O₃, Alkalimetalloxide wie Na₂O oder K₂O und Keimbildner wie P₂O₅. Darüber hinaus können sie als weitere Komponenten beispielsweise weitere Alkalimetalloxide, Erdalkalimetalloxide und ZnO sowie in geringen Mengen färbende und fluoreszierende Metalloxide enthalten.

DE 24 51 121 beschreibt Lithiumdisilikat-Glaskeramiken, die K₂O und Al₂O₃ und P₂O₅ enthalten. Sie werden aus entsprechenden keimhaltigen Ausgangsgläsern hergestellt, die zur Kristallisation von Lithiumdisilikat auf Temperaturen von 850 bis 870 °C erwärmt werden.

EP 2 377 830 A1 beschreibt Lithiumsilikat-Dentalglaskeramik, die 9,0 bis 30,0 Gew.-% Übergangsmetalloxid ausgewählt aus der Gruppe bestehend aus Oxiden von Yttrium, Oxiden von Übergangsmetallen mit Ordnungszahl 41 bis 79 und Mischungen dieser Oxide enthält. Diese zeichnet sich durch einen hohen Brechungsindex bei sehr guten mechanischen und optischen Eigenschaften aus. Die Dentalglaskeramik enthält als weitere Kristallphasen KAlSiO₄ und LaPO₄. Färbe- und/oder Fluoreszenzmittel, z.B. CeO₂, Er₂O₃ oder Tb₄O₇, können ebenfalls vorhanden sein.

WO 2013/053868 A2 betrifft Lithiumsilikat-Glaskeramik, die sechswertiges Metalloxid ausgewählt aus MoO₃, WO₃ und Mischungen davon in einer Menge von 0,1 bis 8,4 Gew.-% enthält und sich für den Einsatz in der Zahnheilkunde eignet. Neben Lithiumsilikat können in der Glaskeramik Lithiumorthophosphat und Quarz als weitere kristalline Phasen vorliegen.

EP 3 150 563 A1 und WO 2015/173394 A1 beschreiben Glaskeramiken, die Lithiumsilikat und Ca-Scheelit enthalten.

Bekannte Glaskeramiken auf Basis von Lithiumsilikat sind aufgrund ihrer ästhetisch gewünschten optischen Anpassung an das sie umgebende natürliche Zahnmaterial naturgemäß nur schwer von diesem zu unterscheiden. Das gilt auch für bekannte fluoreszierende Glaskeramiken, da die ihnen zugegebenen Fluoreszenzmittel dazu dienen, die Fluoreszenz des natürlichen Zahnmaterials in hohem Maße zu imitieren.

Der Erfindung liegt daher die Aufgabe zugrunde, eine Lithiumsilikat-Glaskeramik zur Verfügung zu stellen, die als Dentalmaterial eingesetzt und bei Bedarf besonders leicht vom sie umgebenden natürlichen Zahnmaterial unterschieden werden kann. Die Glaskeramik soll für die Verwendung als restauratives Dentalmaterial mit vorteilhaften mechanischen und optischen Eigenschaften und für die einfache Verarbeitung zu dentalen Restaurationen geeignet sein.

Diese Aufgabe wird durch die Lithiumsilikat-Glaskeramik nach einem der Ansprüche 1 bis 16 gelöst. Gegenstand der Erfindung sind ebenfalls das Ausgangsglas mit Keimen nach Anspruch 17, das Verfahren zur Herstellung der Glaskeramik nach den Ansprüchen 18 und 19 sowie die Verwendung der Glaskeramik und des Ausgangsglases mit Keimen nach Anspruch 20.

Die erfindungsgemäße Lithiumsilikat-Glaskeramik zeichnet sich dadurch aus, dass sie 0 bis 6,0 Gew.-% CaO enthält und Lithiumsilikat als Hauptkristallphase und Scheelit in Form von Ca-Scheelit (CaWO₄) und/oder Sr-Scheelit (SrWO₄) und/oder Powellit in Form von Ca-Powellit (CaMoO₄) und/oder Sr-Powellit (SrMoO₄) als weitere Kristallphasen enthält. Mithin enthält die Glaskeramik Scheelit, Powellit oder eine Mischung von diesen als weitere Kristallphase(n).

Überraschenderweise fluoresziert die erfindungsgemäße Lithiumsilikat-Glaskeramik bei einer Anregung mit UV-Licht einer Wellenlänge von 254 nm. Die Fluoreszenzeigenschaften werden hierbei durch die enthaltenen Scheelit- und/oder Powellit-Kristalle erzielt, wobei Scheelit eine blauweiße und Powellit eine grüngelbe Fluoreszenz zeigt. Diese Fluoreszenz kann z.B. vorteilhaft verwendet werden, um dentale Restaurationen aus der erfindungsgemäßen Lithiumsilikat-Glaskeramik von natürlichem Zahnmaterial klar unterscheidbar zu machen.

Ebenso ist es sehr überraschend, dass die erfindungsgemäße Lithiumsilikat-Glaskeramik durch die doppelte gesteuerte Kristallisation der Kristallphasen von einerseits Lithiumsilikat und andererseits Scheelit und/oder Powellit gebildet werden kann. Durch gezielte Keimbildung und Kristallisation können die genannten Kristallphasen aus entsprechenden Ausgangsgläsern erzeugt werden. Dabei laufen Keimbildung und Wachstum beider Kristallphasen in einem homogenen, nichtphasenseparierten Ausgangsglas offenbar als parallele Festkörperreaktionen ab.

Auch hat sich überraschenderweise gezeigt, dass die Glaskeramik eine Kombination von wünschenswerten optischen und mechanischen Eigenschaften sowie Verarbeitungseigenschaften besitzt, die für den Einsatz als Dentalmaterial vorteilhaft sind. Insbesondere zeichnet sie eine hohe Festigkeit, eine hohe Röntgenopazität und Fluoreszenz bei Anregung mit fernem UV-Licht aus. Es war weiterhin nicht zu erwarten, dass durch das Vorsehen von Scheelit und/oder Powellit als weiterer Kristallphase neben Lithiumsilikat als Hauptkristallphase dennoch sehr gute optische Eigenschaften erzielt werden können. Denn viele Nebenkristallphasen haben einen negativen Effekt auf die optischen Eigenschaften von Lithiumsilikat-Glaskeramiken. Sie können beispielsweise die Transluzenz vermindern und sie können ebenfalls die Einfärbbarkeit der Glaskeramik beeinträchtigen, was bei der Nachahmung der Farbe des zu ersetzenden natürlichen Zahnmaterials zu erheblichen Schwierigkeiten führen kann.

Die erfindungsgemäße Lithiumsilikat-Glaskeramik enthält vorzugsweise 51,0 bis 77,0, insbesondere 55,0 bis 75,0 und bevorzugt 64,0 bis 74,0 Gew.-% SiO₂.

Auch ist es bevorzugt, dass die Lithiumsilikat-Glaskeramik 8,0 bis 20,0, insbesondere 11,0 bis 17,0 und besonders bevorzugt 13,0 bis 15,0 Gew.-% Li₂O enthält.

Eine Lithiumsilikat-Glaskeramik ist bevorzugt, bei der die kombinierte Menge an CaO und SrO 0,1 bis 10,0, bevorzugt 0,5 bis 7,0 und besonders bevorzugt 1,0 bis 5,0 Gew.-% beträgt.

Weiter ist eine Lithiumsilikat-Glaskeramik bevorzugt, die 0,1 bis 4,0 und besonders bevorzugt 0,5 bis 2,0 Gew.-% CaO enthält.

In einer bevorzugten Ausführungsform enthält die erfindungsgemäße Lithiumsilikat-Glaskeramik 0 bis 10,0, insbesondere 0,1 bis 7,0 und bevorzugt 0,5 bis 4,0 Gew.-% SrO.

Auch ist eine Lithiumsilikat-Glaskeramik bevorzugt, die 0 bis 12,0, insbesondere 1,0 bis 8,0 und bevorzugt 3,0 bis 5,0 Gew.-% MoO₃ enthält.

In einer weiteren bevorzugten Ausführungsform enthält die Lithiumsilikat-Glaskeramik 0 bis 22,0, insbesondere 1,0 bis 14,0 und bevorzugt 4,0 bis 7,0 Gew.-% WO₃.

Des Weiteren ist eine erfindungsgemäße Lithiumsilikat-Glaskeramik bevorzugt, die 1,5 bis 6,0, insbesondere 2,0 bis 5,0 und bevorzugt 2,5 bis 4,5 Gew.-% P₂O₅ enthält. Es wird angenommen, dass P₂O₅ als Keimbildner wirkt.

Auch ist es bevorzugt, dass die Lithiumsilikat-Glaskeramik neben Li₂O weiteres Alkalimetalloxid Me^{I}₂O in einer Menge von 0 bis 6,0, insbesondere 1,0 bis 5,0 und besonders bevorzugt 1,5 bis 4,0 Gew.-% enthält. Der Begriff "weiteres Alkalimetalloxid Me^{I}₂O" bezeichnet Alkalimetalloxid mit Ausnahme von Li₂O, wobei dieses weitere Oxid Me^{I}₂O insbesondere ausgewählt ist aus Na₂O und K₂O. Besonders bevorzugt enthält die Lithiumsilikat-Glaskeramik mindestens eines und insbesondere alle der folgenden weiteren Alkalimetalloxide Me^{I}₂O in den angegebenen Mengen:

| Komponente | Gew.-% |
|---|---|
| Na₂O | 0 - 6,0, insbesondere 0,1 bis 6,0 |
| K₂O | 0 - 4,5, insbesondere 0,1 bis 4,5 |

Des Weiteren ist es bevorzugt, dass die Lithiumsilikat-Glaskeramik neben CaO und SrO weiteres Oxid zweiwertiger Elemente Me^{II}O in einer Menge von 0 bis 4,0 und insbesondere 1,0 bis 3,5 Gew.-% enthält, wobei dieses weitere Oxid Me^{II}O insbesondere ausgewählt ist aus MgO und ZnO. Besonders bevorzugt enthält die Lithiumsilikat-Glaskeramik mindestens eines und insbesondere alle der folgenden weiteren Oxide zweiwertiger Elemente Me^{II}O in den angegebenen Mengen:

| Komponente | Gew.-% |
|---|---|
| MgO | 0 - 4,0, insbesondere 0,1 bis 4,0 |
| ZnO | 0 - 4,0, insbesondere 0,1 bis 4,0 |

Es ist weiter eine erfindungsgemäße Lithiumsilikat-Glaskeramik bevorzugt, die 0 bis 11,0 und insbesondere 1,0 bis 9,0 Gew.-% Oxid dreiwertiger Elemente Me^{III}₂O₃ enthält, wobei dieses Oxid Me^{III}₂O₃ insbesondere ausgewählt ist aus Al₂O₃, B₂O₃, Y₂O₃, La₂O₃ und Er₂O₃. Besonders bevorzugt enthält die Lithiumsilikat-Glaskeramik mindestens eines und insbesondere alle der folgenden Oxide dreiwertiger Elemente Me^{III}₂O₃ in den angegebenen Mengen:

| Komponente | Gew.-% |
|---|---|
| Al₂O₃ | 0 - 11,0, insbesondere 0,1 bis 11,0, bevorzugt 1,0 bis 5,0 |
| B₂O₃ | 0 - 7,0, insbesondere 0,1 bis 7,0 |
| Y₂O₃ | 0 - 10,0, insbesondere 0,1 bis 10,0 |
| La₂O₃ | 0 - 3,0, insbesondere 0,1 bis 3,0 |
| Er₂O₃ | 0 - 3,5, insbesondere 0,1 bis 3,5 |

Ferner ist eine Lithiumsilikat-Glaskeramik bevorzugt, die weiteres Oxid vierwertiger Elemente Me^{IV}O₂ in einer Menge von 0 bis 11,0 Gew.-% enthält. Der Begriff "weiteres Oxid vierwertiger Elemente Me^{IV}O₂" bezeichnet vierwertige Oxide mit Ausnahme von SiO₂, wobei dieses weitere Oxid Me^{IV}O₂ insbesondere ausgewählt ist aus ZrO₂, GeO₂, MnO₂ und SnO₂. Besonders bevorzugt enthält die Lithiumsilikat-Glaskeramik mindestens eines und insbesondere alle der folgenden weiteren Oxide vierwertiger Elemente Me^{IV}O₂ in den angegebenen Mengen:

| Komponente | Gew.-% |
|---|---|
| ZrO₂ | 0 - 11,0, insbesondere 0,1 bis 11,0 |
| GeO₂ | 0 - 9,5, insbesondere 0,1 bis 9,5 |
| SnO₂ | 0 - 7,0, insbesondere 0,1 bis 7,0 |
| MnO₂ | 0 - 2,5, insbesondere 0,1 bis 2,5 |

Außerdem ist eine Lithiumsilikat-Glaskeramik bevorzugt, die weiteres Oxid fünfwertiger Elemente Me^{V}₂O₅ in einer Menge von 0 bis 11,5 und insbesondere 1,0 bis 5,0 Gew.-% enthält. Der Begriff "weiteres Oxid fünfwertiger Elemente Me^{V}₂O₅" bezeichnet fünfwertige Oxide mit Ausnahme von P₂O₅, wobei dieses weitere Oxid Me^{V}₂O₅ insbesondere ausgewählt ist aus V₂O₅, Ta₂O₅ und Nb₂O₅. Besonders bevorzugt enthält die Lithiumsilikat-Glaskeramik mindestens eines und insbesondere alle der folgenden weiteren Oxide fünfwertiger Elemente Me^{V}₂O₅ in den angegebenen Mengen:

| Komponente | Gew.-% |
|---|---|
| V₂O₅ | 0 - 2,5, insbesondere 0,1 bis 2,5 |
| Ta₂O₅ | 0 - 2,0, insbesondere 0,1 bis 2,0 |
| Nb₂O₅ | 0 - 11,5, insbesondere 0,1 bis 11,5 |

Weiterhin ist eine erfindungsgemäße Lithiumsilikat-Glaskeramik bevorzugt, die 0 bis 2,0 und insbesondere 0,1 bis 1,0 Gew.-% Fluor enthält.

Besonders ist eine erfindungsgemäße Lithiumsilikat-Glaskeramik bevorzugt, die unabhängig voneinander mindestens eine und bevorzugt alle folgenden Komponenten in den angegebenen Mengen enthält:

| Komponente | Gew.-% |
|---|---|
| SiO₂ | 51,0 - 77,0 |
| Li₂O | 8,0 - 20,0 |
| SrO | 0 - 10,0 |
| MoO₃ | 0 - 12,0 |
| WO₃ | 0 - 22,0 |
| Me^{I}₂O | 0 - 6,0 |
| Me^{II}O | 0 - 4,0 |
| Me^{III}₂O₃ | 0 - 11,0 |
| Me^{IV}O₂ | 0 - 11,0 |
| P₂O₅ | 1,5 - 6,0 |
| Me^{V}₂O₅ | 0 - 11,5 |
| Fluor | 0 - 2,0 |

wobei Me^{I}₂O, Me^{II}O, Me^{III}₂O₃, Me^{IV}O₂ und Me^{V}₂O₅ die oben angegebene Bedeutung haben.

Die Eigenschaften der Lithiumsilikat-Glaskeramik werden maßgeblich durch die Kristallphasen beeinflusst. Die erfindungsgemäße Glaskeramik enthält Lithiumsilikat als Hauptkristallphase. Der Begriff "Lithiumsilikat" bezeichnet mindestens eine Kristallphase ausgewählt aus Lithiumdisilikat und Lithiummetasilikat. Mithin enthält die erfindungsgemäße Glaskeramik Lithiumdisilikat, Lithiummetasilikat oder eine Mischung von Lithiumdisilikat und Lithiummetasilikat als Hauptkristallphase. In einer bevorzugten Ausführungsform enthält die erfindungsgemäße Glaskeramik Lithiumdisilikat als Hauptkristallphase.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Die erfindungsgemäße Glaskeramik enthält zusätzlich zu Lithiumsilikat als Hauptkristallphase noch Scheelit und/oder Powellit als weitere Kristallphasen. Scheelit liegt hierbei in Form von Ca-Scheelit (CaWO₄) und/oder Sr-Scheelit (SrWO₄) und Powellit in Form von Ca-Powellit (CaMoO₄) und/oder Sr-Powellit (SrMoO₄) vor.

Die erfindungsgemäße Glaskeramik kann ferner weitere Kristallphasen, wie beispielsweise Li₃PO₄ und/oder SiO₂-Modifikationen, enthalten.

Die Art und die Menge der gebildeten Kristallphasen können insbesondere durch die Zusammensetzung des Ausgangsglases sowie das Verfahren zur Herstellung der Glaskeramik gesteuert werden. Die Beispiele veranschaulichen dies anhand der Variation der Zusammensetzung und des Herstellungsverfahrens.

Die in der Lithiumsilikat-Glaskeramik enthaltenen Scheelit-und Powellit-Kristallphasen sind aufgrund ihrer Zusammensetzung auch röntgenopak. Dies führt zu einem vorteilhaften Kontrast zwischen natürlichem und künstlichem Material auf Röntgenaufnahmen, wie sie insbesondere im Dentalbereich verwendet werden. Die erfindungsgemäße Lithiumsilikat-Glaskeramik weist eine Röntgenopazität gemäß EN ISO 4049 von insbesondere mehr als 120%, bevorzugt mehr als 150% und besonders bevorzugt mehr als 180% auf.

Die erfindungsgemäße Lithiumsilikat-Glaskeramik weist zudem eine biaxiale Bruchfestigkeit σ_{Biax} von vorzugsweise mindestens 300 MPa, insbesondere mehr als 350 und bevorzugt mehr als 400 MPa auf. Die biaxiale Bruchfestigkeit wurde gemäß ISO 6872 (2015) (Kolben-auf-drei-Kugeln-Prüfung) bestimmt.

Auch ist es bevorzugt, dass die Lithiumsilikat-Glaskeramik einen thermischen Wärmeausdehnungskoeffizienten WAK gemäß ISO 6872 (2015) (gemessen im Bereich von 100 bis 500 °C) von 9,5 bis 11,0 ^{∗} 10⁻⁶ K⁻¹ aufweist. Eine Einstellung des Wärmeausdehnungskoeffizienten auf einen gewünschten Wert erfolgt insbesondere durch die Art und Menge der in der Glaskeramik vorhandenen Kristallphasen sowie die chemische Zusammensetzung der Glaskeramik.

In einer Ausführungsform beträgt die Helligkeit L gemäß EN ISO 11664-4 der Lithiumsilikat-Glaskeramik bevorzugt mehr als 70, insbesondere mehr als 80 und besonders bevorzugt mehr als 85.

In einer weiteren Ausführungsform weist die Lithiumsilikat-Glaskeramik eine Transluzenz, gemessen als Kontrastwert (CR) gemäß British Standard BS 5612, von bevorzugt mehr als 60, insbesondere mehr als 65 und besonders bevorzugt mehr als 75 auf.

Mithin bietet die erfindungsgemäße Lithiumsilikat-Glaskeramik, eine wünschenswerte Kombination von vorteilhaften optischen und mechanischen Eigenschaften, wie sie insbesondere für ein Dentalmaterial angestrebt wird.

Ebenfalls werden verschiedene Vorstufen mit entsprechender Zusammensetzung offenbart, aus denen die erfindungsgemäße Lithiumsilikat-Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechender Zusammensetzung" bedeutet, dass diese Vorstufen die gleichen Komponenten in den gleichen Mengen wie die Lithiumsilikat-Glaskeramik enthalten, wobei die Komponenten mit Ausnahme von Fluor als Oxide berechnet werden, so wie es bei Gläsern und Glaskeramiken üblich ist.

Offenbart wird daher ebenfalls ein Ausgangsglas, das die Komponenten der erfindungsgemäßen Lithiumsilikat-Glaskeramik enthält. Das Ausgangsglas enthält daher insbesondere geeignete Mengen an SiO₂, Li₂O sowie WO₃ und/oder MoO₃, die zur Ausbildung der erfindungsgemäßen Glaskeramik mit Lithiumsilikat als Hauptkristallphase sowie Scheelit und/oder Powellit als weiterer Kristallphase erforderlich sind. Weiter kann das Ausgangsglas auch noch andere Komponenten, insbesondere CaO und/oder SrO, enthalten, wie sie oben für die erfindungsgemäße Lithiumsilikat-Glaskeramik angegeben sind. Es sind alle solche Ausführungsformen für die Komponenten des Ausgangsglases bevorzugt, die auch für die Komponenten der erfindungsgemäßen Lithiumsilikat-Glaskeramik als bevorzugt angegeben sind.

Offenbart wird weiter auch ein solches Ausgangsglas, das Keime für die Kristallisation von Lithiummetasilikat, Lithiumdisilikat, Scheelit und/oder Powellit enthält. Insbesondere betrifft die Erfindung auch ein Ausgangsglas, das 0 bis 6,0 Gew.-% CaO und 8,0 bis 20,0 Gew.-% Li₂O und gegebenenfalls weitere Komponenten der erfindungsgemäßen Lithiumsilikat-Glaskeramik enthält und Keime für die Kristallisation von Scheelit in Form von Ca-Scheelit (CaWO₄) und/oder Sr-Scheelit (SrWO₄) und/oder für die Kristallisation von Powellit in Form von Ca-Powellit (CaMoO₄) und/oder Sr-Powellit (SrMoO₄) enthält.

Durch Wärmebehandlung des Ausgangsglases kann zunächst die weitere Vorstufe Ausgangsglas mit Keimen erzeugt werden. Durch Wärmebehandlung dieser weiteren Vorstufe kann dann die erfindungsgemäße Lithiumsilikat-Glaskeramik erzeugt werden. Es ist bevorzugt, die erfindungsgemäße Lithiumsilikat-Glaskeramik durch Wärmebehandlung des Ausgangsglases mit Keimen zu bilden.

Es ist bevorzugt, das Ausgangsglas einer Wärmebehandlung bei einer Temperatur von 440 bis 550 °C, insbesondere 460 bis 530 °C, für eine Dauer von bevorzugt 5 bis 60 min, insbesondere 10 bis 30 min, zu unterwerfen, um das Ausgangsglas mit Keimen für die Kristallisation von Lithiummetasilikat, Lithiumdisilikat, Scheelit und/oder Powellit zu erzeugen.

Es ist weiter bevorzugt, das Ausgangsglas mit Keimen einer Wärmebehandlung bei einer Temperatur von 550 bis 940 °C, insbesondere 580 bis 920 °C für eine Dauer von 5 bis 90 min, bevorzugt 10 bis 60 min, zu unterwerfen, um die Lithiumsilikat-Glaskeramik herzustellen.

Die Erfindung betrifft ebenfalls ein Verfahren zur Herstellung der erfindungsgemäßen Lithiumsilikat-Glaskeramik, bei dem das Ausgangsglas mit den Komponenten der Lithiumsilikat-Glaskeramik oder das Ausgangsglas mit Keimen mit den Komponenten der Lithiumsilikat-Glaskeramik mindestens einer Wärmebehandlung im Bereich von 550 bis 940 °C für eine Dauer von insbesondere 5 bis 90 min, bevorzugt 10 bis 60 min und besonders bevorzugt 10 bis 30 min unterzogen wird.

Das Ausgangsglas und das Ausgangsglas mit Keimen können z.B. in Form eines Massivglasrohlings, eines Pulverpresslings oder eines Pulvers der mindestens einen Wärmebehandlung unterzogen werden.

Die im erfindungsgemäßen Verfahren durchgeführte mindestens eine Wärmebehandlung kann auch im Rahmen eines Heißpressens oder Aufsinterns des Ausgangsglases oder des Ausgangsglases mit Keimen erfolgen.

In einer bevorzugten Ausführungsform umfasst das erfindungsgemäße Verfahren
(a) die Wärmebehandlung des Ausgangsglases bei einer Temperatur von 460 bis 530 °C für eine Dauer von 10 bis 30 min, um das Ausgangsglas mit Keimen zu bilden, und
(b) die Wärmebehandlung des Ausgangsglases mit Keimen bei einer Temperatur von 580 bis 920 °C für eine Dauer von 10 bis 60 min, um die Lithiumsilikat-Glaskeramik zu bilden.

Die Herstellung des Ausgangsglases erfolgt insbesondere in der Weise, dass eine Mischung von geeigneten Ausgangsmaterialien, wie z.B. Carbonaten, Oxiden, und Phosphaten, bei Temperaturen von insbesondere 1300 bis 1700 °C, bevorzugt bei 1500 bis 1600 °C, für eine Dauer von 0,5 bis 3 h, bevorzugt 1 bis 3 h erschmolzen wird. Zur Erzielung einer besonders hohen Homogenität kann die erhaltene Glasschmelze in Wasser gegossen werden, um ein Glasgranulat zu bilden, und das erhaltene Granulat kann dann erneut aufgeschmolzen werden.

Die Schmelze kann dann in Formen gegossen werden, um Rohlinge des Ausgangsglases, sogenannte Massivglasrohlinge oder monolithische Rohlinge, zu erzeugen. Vorzugsweise erfolgt die Abkühlung ab einer Temperatur von 440 bis 550 °C mit einer Abkühlgeschwindigkeit von 2 bis 3 K/min bis auf Raumtemperatur. Dies ist insbesondere zur Erzeugung spannungsfreier Glasprodukte vorteilhaft.

Es ist ebenfalls möglich, die Schmelze erneut in Wasser zu geben, um ein Granulat herzustellen. Dieses Granulat kann nach Mahlen und gegebenenfalls Zugabe weiterer Komponenten zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung unterzogen. Es ist bevorzugt, dass zunächst eine erste Wärmebehandlung durchgeführt wird, um ein Ausgangsglas mit Keimen herzustellen, welche zur Bildung von Lithiummetasilikat-, Lithiumdisilikat-, Scheelit- und/oder Powellit-Kristallen geeignet sind. Das Glas mit Keimen wird dann üblicherweise mindestens einer weiteren Temperaturbehandlung bei einer höheren Temperatur unterworfen, um Kristallisation von Lithiumsilikat, insbesondere von Lithiumdisilikat, und Scheelit und/oder Powellit zu bewirken.

Die erfindungsgemäßen Lithiumsilikat-Glaskeramiken und die oben beschriebenen Gläser liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Inlays, Onlays, Kronen, Veneers, Schalen oder Abutments, vorliegen.

Aus den erfindungsgemäßen Glaskeramiken und den oben beschriebenen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das oben beschriebene Ausgangsglas und insbesondere das oben beschriebene Ausgangsglas mit Keimen sowie die erfindungsgemäße Lithiumsilikat-Glaskeramik verwendet werden. Dabei können die Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines computergestützten Verfahrens, insbesondere eines CAD/CAM-Verfahrens, durchgeführt wird. Für die maschinelle Bearbeitung können das oben beschriebene Ausgangsglas, das oben beschriebene Ausgangsglas mit Keimen und die erfindungsgemäße Lithiumsilikat-Glaskeramik verwendet werden. Dabei können die Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiumsilikat-Glaskeramik verwendet.

Nach der Herstellung der wunschgemäß geformten dentalen Restauration, z.B. durch Verpressen oder maschinelle Bearbeitung, kann diese noch wärmebehandelt werden, um die Porosität, z.B. eines eingesetzten porösen Pulverpresslings, zu vermindern.

Die erfindungsgemäßen Glaskeramiken und die oben beschriebenen Gläser eignen sich allerdings auch als Beschichtungsmaterial von z.B. Keramiken und Glaskeramiken. Die Erfindung ist daher ebenfalls auf die Verwendung der Gläser oder der erfindungsgemäßen Glaskeramiken zur Beschichtung von insbesondere Keramiken und Glaskeramiken gerichtet.

Die Erfindung betrifft auch ein Verfahren zur Beschichtung von Keramiken und Glaskeramiken, bei dem erfindungsgemäße Glaskeramiken oder Gläser auf die Keramik oder Glaskeramik aufgebracht und erhöhter Temperatur ausgesetzt werden.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird erfindungsgemäße Glaskeramik oder Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges die erfindungsgemäße Glaskeramik mit Lithiumdisilikat als Hauptkristallphase und Scheelit und/oder Powellit als weiterer Kristallphase vorliegt, da eine solche Glaskeramik über besonders gute Eigenschaften verfügt.

Aufgrund der vorstehend geschilderten Eigenschaften der erfindungsgemäßen Lithiumsilikat-Glaskeramik und der oben beschriebenen Gläser als deren Vorläufer eignen sich diese insbesondere auch zum Einsatz in der Zahnheilkunde. Gegenstand der Erfindung ist daher auch die Verwendung der erfindungsgemäßen Glaskeramik oder der erfindungsgemäßen Gläser als Dentalmaterial und insbesondere zur Herstellung dentaler Restaurationen, wie Kronen, Brücken und Abutments.

Die Erfindung wird im Folgenden anhand von Beispielen näher erläutert.

### Beispiele

### Beispiele 1 bis 34 - Zusammensetzung und Kristallphasen

Es wurden insgesamt 34 erfindungsgemäße Glaskeramiken mit der aus Tabelle I angegebenen Zusammensetzung über Erschmelzung entsprechender Ausgangsgläser und anschließende Wärmebehandlung zur gesteuerten Keimbildung und Kristallisation hergestellt.

Die angewendeten Wärmebehandlungen zur gesteuerten Keimbildung und gesteuerten Kristallisation sind ebenfalls in der Tabelle I angegeben. Dabei bedeuten
- T_{g}: Glasübergangstemperatur, bestimmt mittels DSC
- T_{S} und t_{S}: Angewendete Temperatur und Zeit für Erschmelzung des Ausgangsglases
- T_{Kb} und t_{Kb}: Angewendete Temperatur und Zeit für Keimbildung des Ausgangsglases
- T_{C} und t_{C}: Angewendete Temperatur und Zeit für die Kristallisation

Die Ausgangsgläser wurden in einem Platintiegel bei einer Temperatur Tₛ von 1500 bis 1600 °C über eine Dauer tₛ von 1 bis 2 h erschmolzen und danach abgekühlt. Bei einigen Gläsern wurde dieser Schritt wiederholt, um eine höhere Homogenisierung zu erreichen. Danach konnte die Schmelze in Form von z.B. Blöcken gegossen oder auch zur Herstellung von z.B. Glasfritten verwendet werden.

Die erstarrten Gläser wurden in einem Muffelofen, der auf Temperaturen T_{Kb} von 460 bis 530 °C vorgeheizt wurde, überführt, für eine Dauer t_{Kb} von 10 bis 30 Minuten bei dieser Temperatur gehalten und anschließend spannungsfrei abgekühlt.

Anschließend wurden von den Blöcken Scheiben mit den Abmessungen von etwa 13 ^{∗} 13 ^{∗} 2 mm gesägt und in einem Ofen vom Typ Programat (Ivoclar Vivadent AG) bei Temperaturen T_{C} zwischen 580 und 920 °C für jeweils eine Dauer t_{C} von 10 bis 60 min kristallisiert. Das Glas gemäß Beispiel 26 wurde davon abweichend in zwei Stufen bei Temperaturen T_{C} von 600 °C und 830 °C für jeweils eine Dauer t_{C} von 10 Minuten kristallisiert.

Nach der Kristallisation wurden die Oberflächen der Scheiben mittels einer 125 µm Diamantscheibe abgeschliffen, und die Kristallphasen wurden mittels Röntgenbeugungsanalysen (XRD) identifiziert.

Bei Beispiel 18 wurde ein Pulverpressling hergestellt. Hierzu wurde die aus dem Ausgangsglas erhaltene Glasfritte getrocknet und mit einer Schwingmühle RM200 der Firma Retsch GmbH, Haan, Deutschland, auf eine mittlere Korngröße von < 90 µm, bezogen auf die Anzahl der Teilchen, gemahlen. Das gemahlene Glaspulver wurde anschließend uniaxial zu kleinen Zylindern verpresst und diese wurden in einem Ofen vom Typ Programat (Ivoclar Vivadent AG) in zwei Stufen bei der Temperatur T_{Kb} für die Dauer t_{Kb} und bei der Temperatur T_{C} für die Dauer t_{C} keimgebildet, kristallisiert und gesintert. An den so hergestellten Prüfkörpern wurden nach Abschleifen der Oberflächen mit einer 125 µm Diamantscheibe Röntgenbeugungsanalysen (XRD) zur Bestimmung der vorhandenen Kristallphasen durchgeführt.

Für alle gemäß den Beispielen 1 bis 34 hergestellten Lithiumsilikat-Glaskeramiken wurde Fluoreszenz unter einer UV-Lampe (Wellenlänge 254 nm) beobachtet. Glaskeramiken mit Scheelit fluoreszierten weißblau, solche mit Powellit grüngelb.

Zur Bestimmung der biaxialen Bruchfestigkeiten gemäß ISO 6872 (2015) (Kolben-auf-drei-Kugeln-Prüfung) wurden auf die Blöcke der Gläser Halter aufgeklebt und diese wurden anschließend mittels einer CAD/CAM Schleifeinheit (Sirona InLab) bearbeitet. Die schleifende Bearbeitung erfolgte mittels diamantbeschichteter Schleifwerkzeuge. Die so erhaltenen Glasplättchen wurden unter den in der Tabelle I angegebenen Bedingungen kristallisiert und anschließend mittels Diamantscheiben auf eine Dicke von 1,2 ± 0,2 mm poliert. An den so hergestellten Proben wurde anschließend die biaxiale Bruchfestigkeit bestimmt.

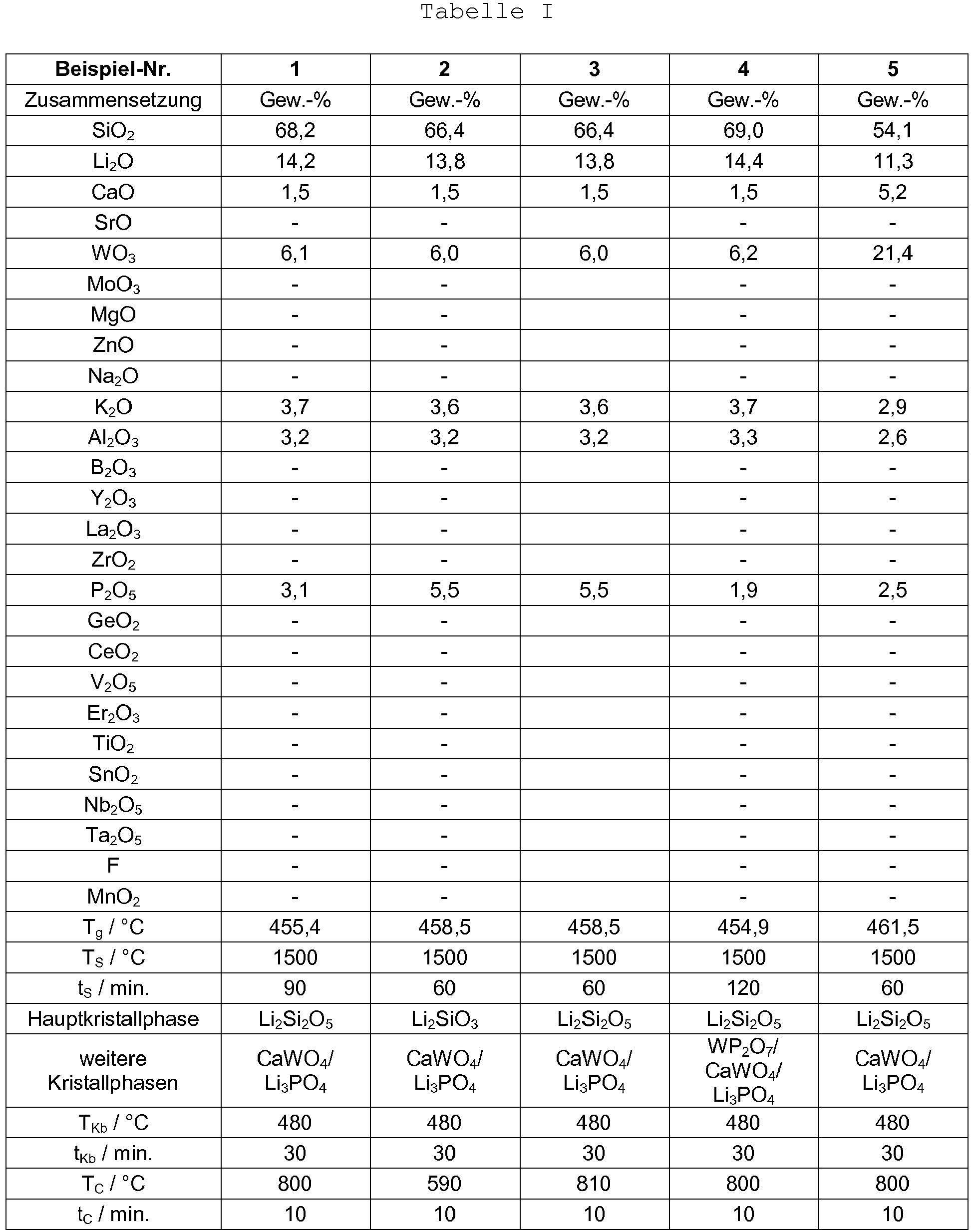

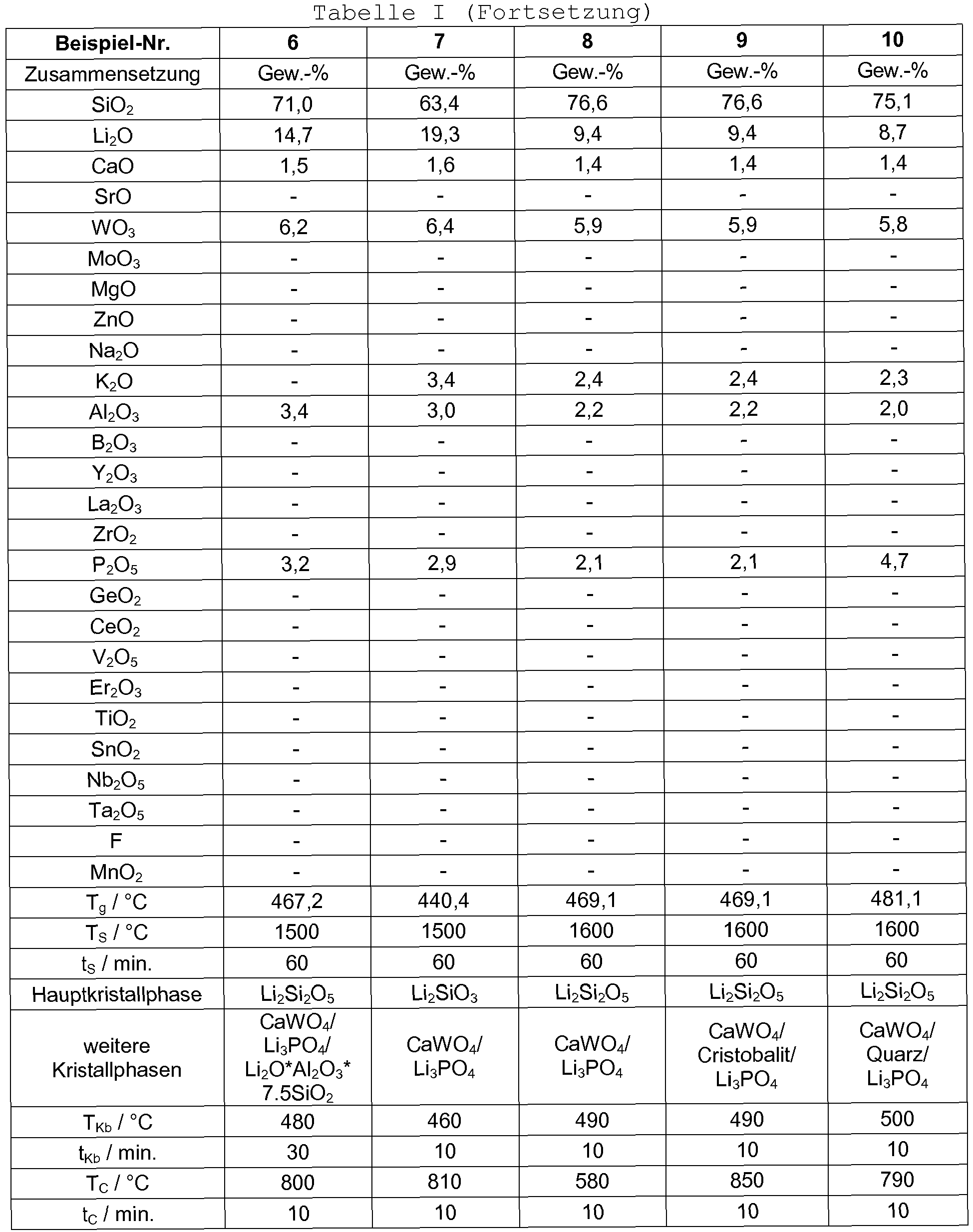

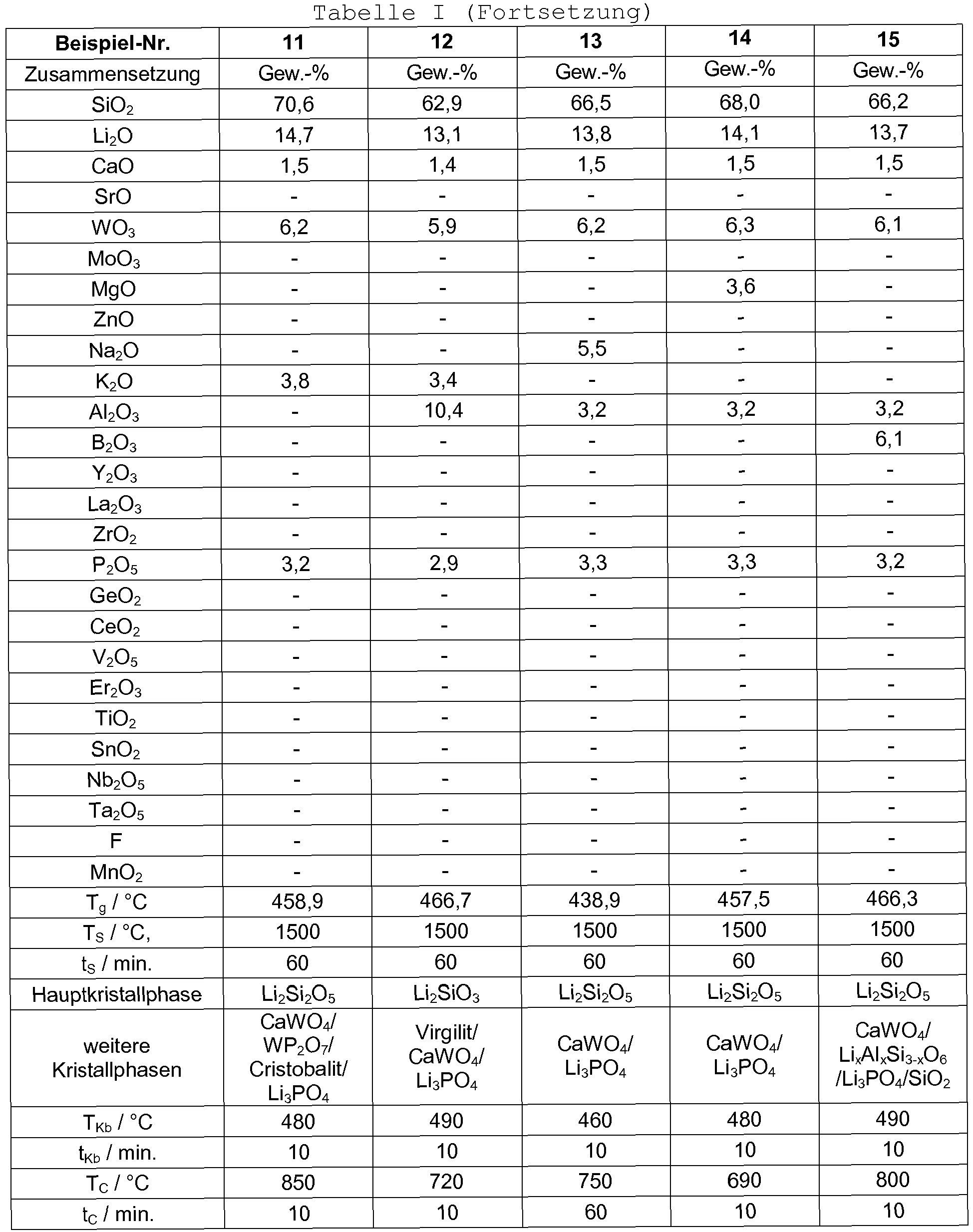

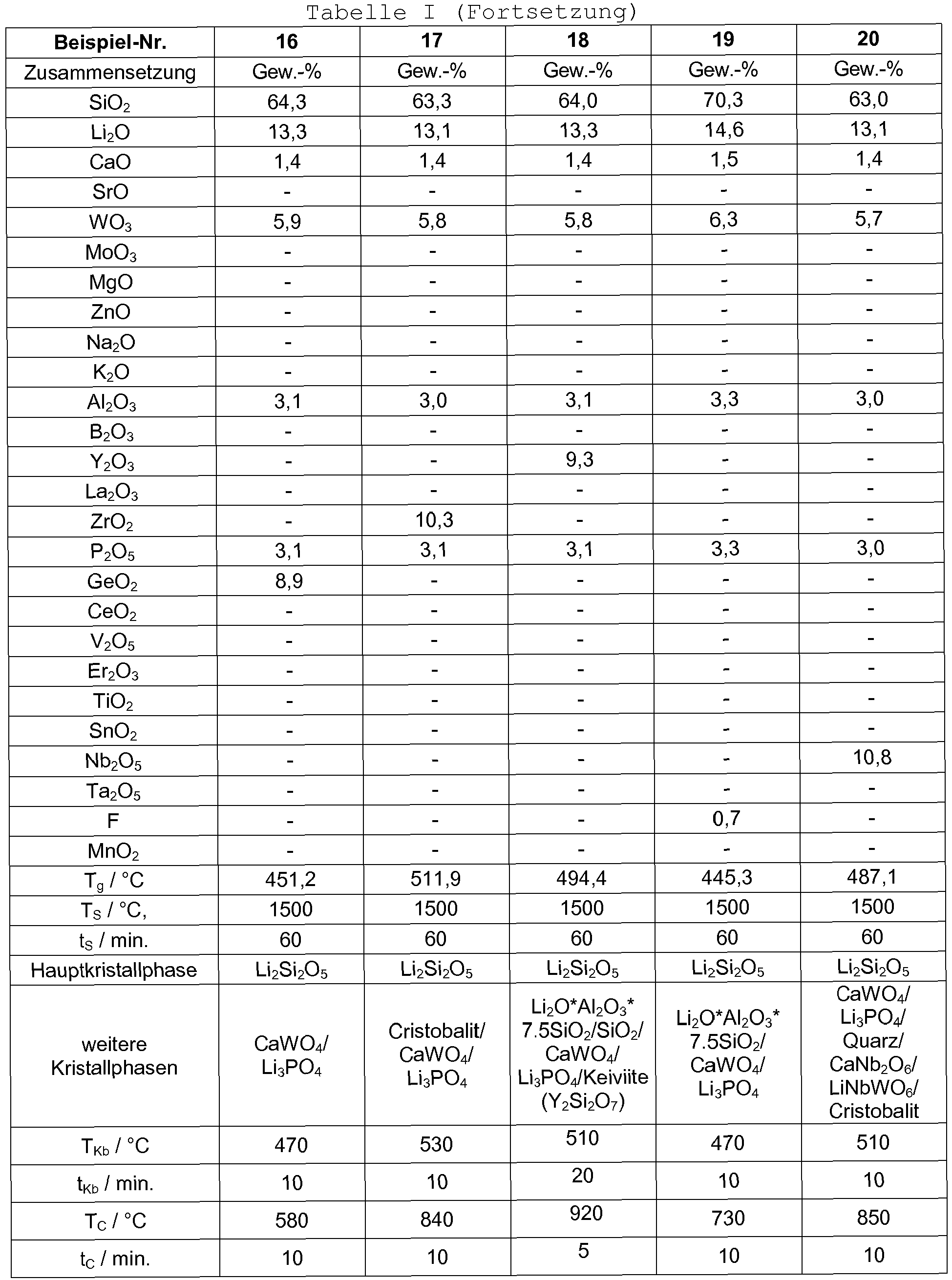

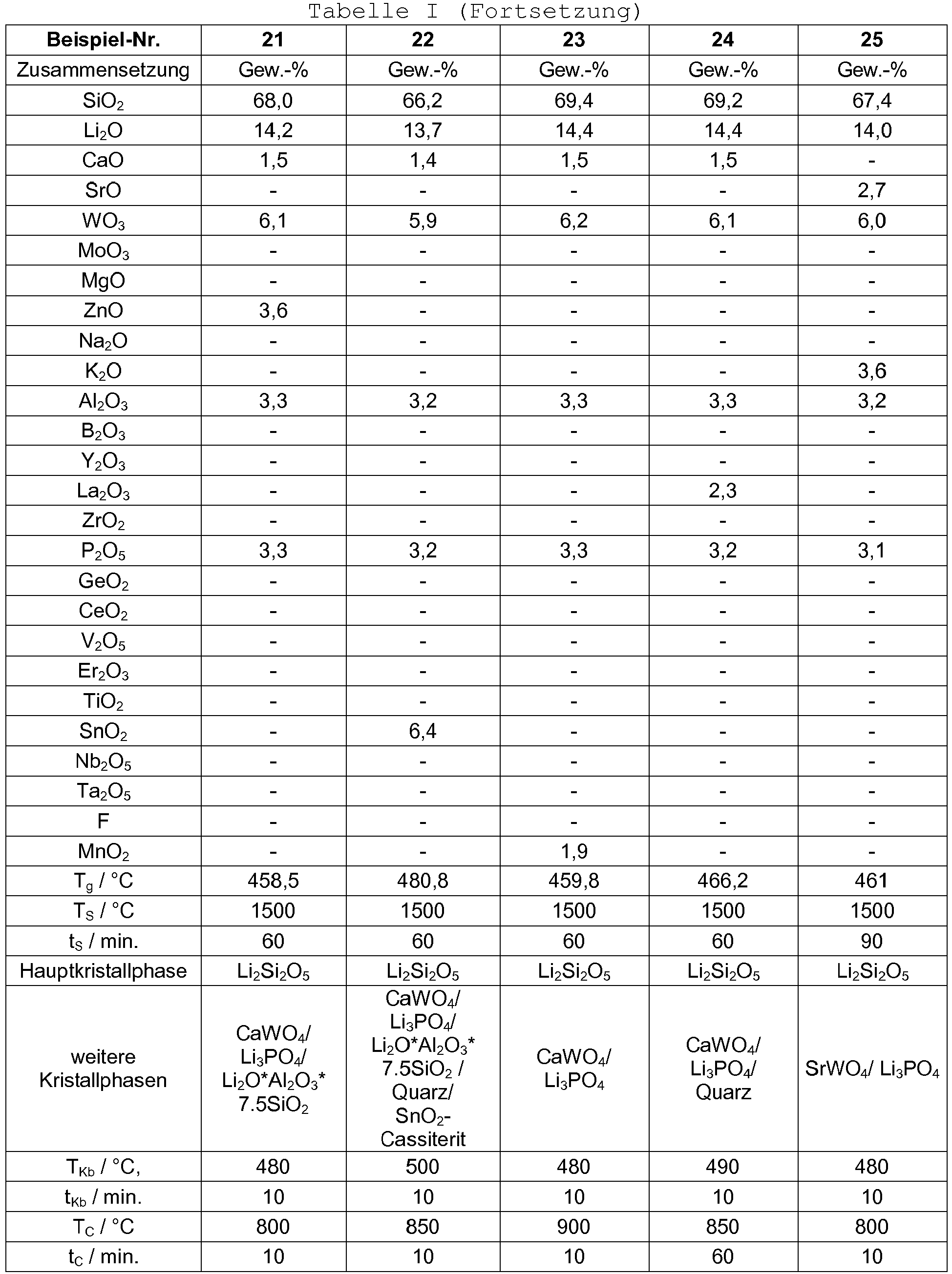

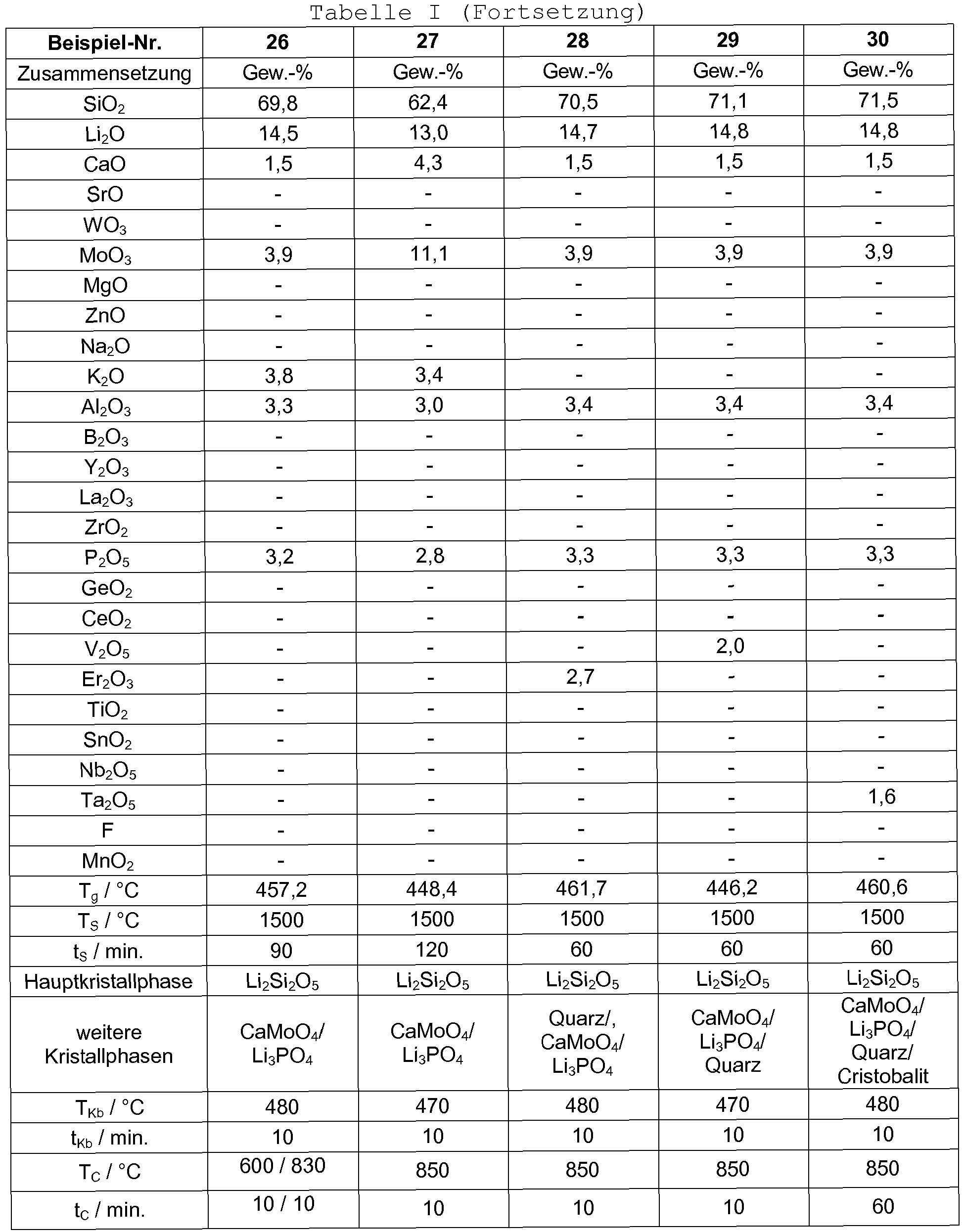

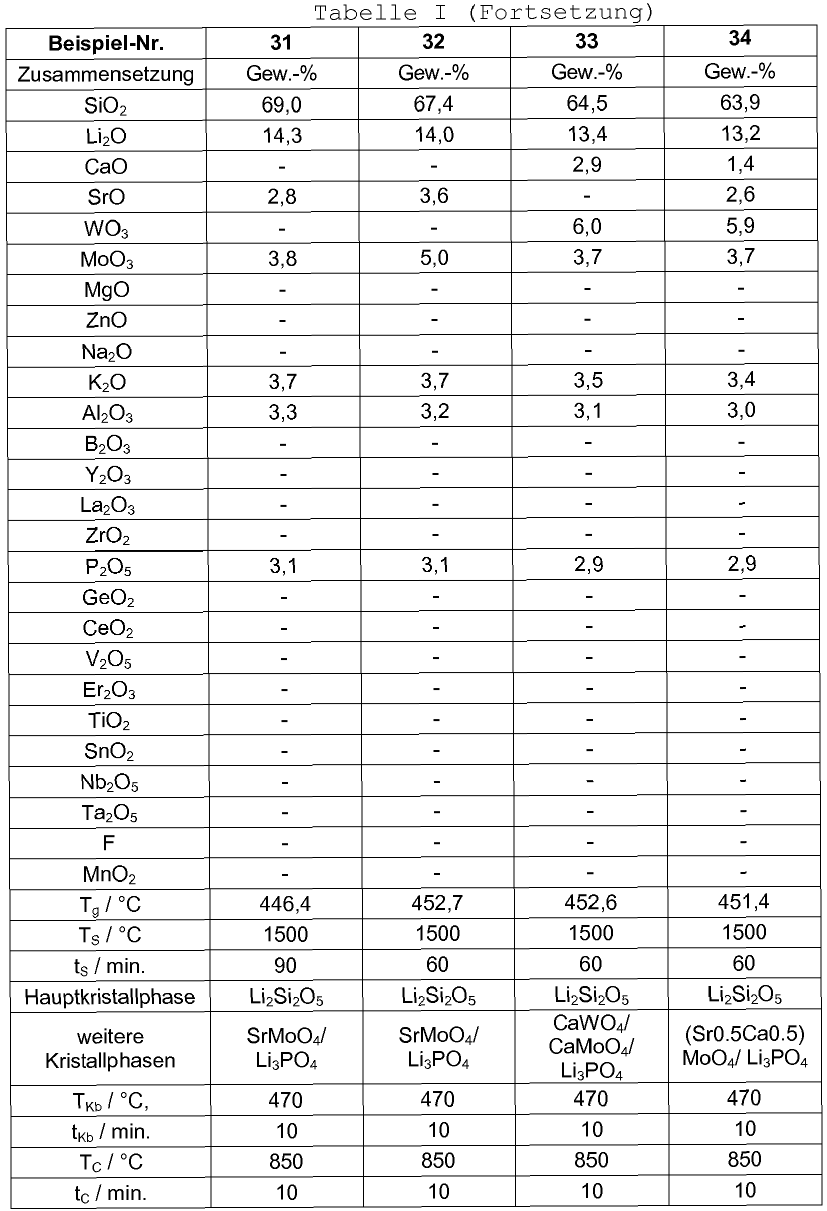

## Patentansprüche

1. Lithiumsilikat-Glaskeramik, die 0 bis 6,0 Gew.-% CaO enthält und Lithiumsilikat als Hauptkristallphase und Scheelit in Form von Ca-Scheelit (CaWO₄) und/oder Sr-Scheelit (SrWO₄) und/oder Powellit in Form von Ca-Powellit (CaMoO₄) und/oder Sr-Powellit (SrMoO₄) als weitere Kristallphasen enthält.

2. Glaskeramik nach Anspruch 1, die 8,0 bis 20,0, insbesondere 11,0 bis 17,0 und bevorzugt 13,0 bis 15,0 Gew.-% Li₂O enthält.

3. Glaskeramik nach Anspruch 1 oder 2, die 51,0 bis 77,0, insbesondere 55,0 bis 75,0 und bevorzugt 64,0 bis 74,0 Gew.-% SiO₂ enthält.

4. Glaskeramik nach einem der Ansprüche 1 bis 3, bei der die kombinierte Menge an CaO und SrO insbesondere 0,1 bis 10,0, bevorzugt 0,5 bis 7,0 und besonders bevorzugt 1,0 bis 5,0 Gew.-% beträgt.

5. Glaskeramik nach einem der Ansprüche 1 bis 4, die 0,1 bis 4,0 und bevorzugt 0,5 bis 2,0 Gew.-% CaO enthält.

6. Glaskeramik nach einem der Ansprüche 1 bis 5, die 0 bis 10,0, insbesondere 0,1 bis 7,0 und bevorzugt 0,5 bis 4,0 Gew.-% SrO enthält.

7. Glaskeramik nach einem der Ansprüche 1 bis 6, die 0 bis 12,0, insbesondere 1,0 bis 8,0 und bevorzugt 3,0 bis 5,0 Gew.-% MoO₃ enthält.

8. Glaskeramik nach einem der Ansprüche 1 bis 7, die 0 bis 22,0, insbesondere 1,0 bis 14,0 und bevorzugt 4,0 bis 7,0 Gew.-% WO₃ enthält.

9. Glaskeramik nach einem der Ansprüche 1 bis 8, die 1,5 bis 6,0, insbesondere 2,0 bis 5,0 und bevorzugt 2,5 bis 4,5 Gew.-% P₂O₅ enthält.

10. Glaskeramik nach einem der Ansprüche 1 bis 9, die mindestens eine und bevorzugt alle folgenden Komponenten in den angegebenen Mengen enthält:
| Komponente | Gew.-% |
|---|---|
| SiO₂ | 51,0 - 77,0 |
| Li₂O | 8,0 - 20,0 |
| SrO | 0 - 10,0 |
| MoO₃ | 0 - 12,0 |
| WO₃ | 0 - 22,0 |
| Me^{I}₂O | 0 - 6,0 |
| Me^{II}O | 0 - 4,0 |
| Me^{III}₂O₃ | 0 - 11,0 |
| Me^{IV}O₂ | 0 - 11,0 |
| P₂O₅ | 1,5 - 6,0 |
| Me^{V}₂O₅ | 0 - 11,5 |
| F | 0 - 2,0, |
wobei
Me^{I}₂O Alkalimetalloxid mit Ausnahme von Li₂O,
Me^{II}O Oxid zweiwertiger Elemente mit Ausnahme von CaO und SrO,
Me^{III}₂O₃ Oxid dreiwertiger Elemente,
Me^{IV}O₂ Oxid vierwertiger Elemente mit Ausnahme von SiO₂ und
Me^{V}₂O₅ Oxid fünfwertiger Elemente mit Ausnahme von P₂O₅ bezeichnet.

11. Glaskeramik nach einem der Ansprüche 1 bis 10, die Lithiumsilikat in Form von Lithiumdisilikat und/oder Lithiummetasilikat enthält.

12. Glaskeramik nach einem der Ansprüche 1 bis 11, die Scheelit in Form von Ca-Scheelit (CaWO₄) und/oder Sr-Scheelit (SrWO₄) enthält.

13. Glaskeramik nach einem der Ansprüche 1 bis 12, die Powellit in Form von Ca-Powellit (CaMoO₄) und/oder Sr-Powellit (SrMoO₄) enthält.

14. Glaskeramik nach einem der Ansprüche 1 bis 13, die bei Anregung mit Licht der Wellenlänge 254 nm fluoresziert.

15. Glaskeramik nach einem der Ansprüche 1 bis 14, die eine Röntgenopazität gemäß EN ISO 4049 von mehr als 120%, insbesondere mehr als 150% und bevorzugt mehr als 180% aufweist.

16. Glaskeramik nach einem der Ansprüche 1 bis 15, die eine biaxiale Bruchfestigkeit gemäß ISO 6872 (2015) (Kolben-auf-drei-Kugeln-Prüfung) von mehr als 300, insbesondere mehr als 350 und bevorzugt mehr als 400 MPa aufweist.

17. Ausgangsglas, das die Komponenten der Glaskeramik nach einem der Ansprüche 2 bis 10 enthält und Keime für die Kristallisation von Scheelit in Form von Ca-Scheelit (CaWO₄) und/oder Sr-Scheelit (SrWO₄) und/oder für die Kristallisation von Powellit in Form von Ca-Powellit (CaMoO₄) und/oder Sr-Powellit (SrMoO₄) enthält.

18. Verfahren zur Herstellung der Glaskeramik gemäß einem der Ansprüche 1 bis 16, bei dem ein Ausgangsglas, das die Komponenten der Glaskeramik enthält, insbesondere das Ausgangsglas mit Keimen gemäß Anspruch 17, mindestens einer Wärmebehandlung im Bereich von 550 bis 940 °C unterzogen wird.

19. Verfahren nach Anspruch 18, bei dem
(a) das Ausgangsglas einer Wärmebehandlung bei einer Temperatur von 460 bis 530 °C für eine Dauer von 10 bis 30 min unterworfen wird, um das Ausgangsglas mit Keimen zu bilden, und
(b) das Ausgangsglas mit Keimen mindestens einer Wärmebehandlung bei einer Temperatur von 580 bis 920 °C für eine Dauer von 10 bis 60 min unterworfen wird, um die Lithiumsilikat-Glaskeramik zu bilden.

20. Verwendung der Glaskeramik gemäß einem der Ansprüche 1 bis 16 oder des Ausgangsglases mit Keimen gemäß Anspruch 17 als Dentalmaterial, insbesondere zur Herstellung dentaler Restaurationen.

## Claims

1. Lithium silicate glass ceramic which comprises lithium silicate as main crystal phase and scheelite in the form of Ca scheelite (CaWO₄) and/or Sr scheelite (SrWO₄) and/or powellite in the form of Ca powellite (CaMoO₄) and/or Sr powellite (SrMoO₄) as further crystal phases.

2. Glass ceramic according to claim 1, which comprises 8.0 to 20.0, in particular 11.0 to 17.0 and preferably 13.0 to 15.0 wt.-% Li₂O.

3. Glass ceramic according to claim 1 or 2, which comprises 51.0 to 77.0, in particular 55.0 to 75.0 and preferably 64.0 to 74.0 wt.-% SiO₂.

4. Glass ceramic according to any one of claims 1 to 3, wherein the combined amount of CaO and SrO is in particular 0.1 to 10.0, preferably 0.5 to 7.0 and particularly preferably 1.0 to 5.0 wt.-%.

5. Glass ceramic according to any one of claims 1 to 4, which comprises 0.1 to 4.0 and preferably 0.5 to 2.0 wt.-% CaO.

6. Glass ceramic according to any one of claims 1 to 5, which comprises 0 to 10.0, in particular 0.1 to 7.0 and preferably 0.5 to 4.0 wt.-% SrO.

7. Glass ceramic according to any one of claims 1 to 6, which comprises 0 to 12.0, in particular 1.0 to 8.0 and preferably 3.0 to 5.0 wt.-% MoO₃.

8. Glass ceramic according to any one of claims 1 to 7, which comprises 0 to 22.0, in particular 1.0 to 14.0 and preferably 4.0 to 7.0 wt.-% WO₃.

9. Glass ceramic according to any one of claims 1 to 8, which comprises 1.5 to 6.0, in particular 2.0 to 5.0 and preferably 2.5 to 4.5 wt.-% P₂O₅.

10. Glass ceramic according to any one of claims 1 to 9, which comprises at least one and preferably all the following components in the amounts specified:
| Component | wt.-% |
|---|---|
| SiO₂ | 51.0 - 77.0 |
| Li₂O | 8.0 - 20.0 |
| SrO | 0 - 10.0 |
| MoO₃ | 0 - 12.0 |
| WO₃ | 0 - 22.0 |
| Me^{I}₂O | 0 - 6.0 |
| Me^{II}O | 0 - 4.0 |
| Me^{III}₂O₃ | 0 - 11.0 |
| Me^{IV}O₂ | 0 - 11.0 |
| P₂O₅ | 1.5 - 6.0 |
| Me^{V}₂O₅ | 0 - 11.5 |
| F | 0 - 2.0, |
wherein
Me^{I}₂O denotes alkali metal oxide with the exception of Li₂O,
Me^{II}O denotes oxide of divalent elements with the exception of CaO and SrO,
Me^{III}₂O₃ denotes oxide of trivalent elements,
Me^{IV}O₂ denotes oxide of tetravalent elements with the exception of SiO₂ and
Me^{V}₂O₅ denotes oxide of pentavalent elements with the exception of P₂O₅.

11. Glass ceramic according to any one of claims 1 to 10, which comprises lithium silicate in the form of lithium disilicate and/or lithium metasilicate.

12. Glass ceramic according to any one of claims 1 to 11, which comprises scheelite in the form of Ca scheelite (CaWO₄) and/or Sr scheelite (SrWO₄).

13. Glass ceramic according to any one of claims 1 to 12, which comprises powellite in the form of Ca powellite (CaMoO₄) and/or Sr powellite (SrMoO₄).

14. Glass ceramic according to any one of claims 1 to 13, which fluoresces when excited by light of the wavelength 254 nm.

15. Glass ceramic according to any one of claims 1 to 14, which has a radiopacity according to EN ISO 4049 of more than 120%, in particular more than 150% and preferably more than 180%.

16. Glass ceramic according to any one of claims 1 to 15, which has a biaxial flexural strength according to ISO 6872 (2015) (piston-on-three-ball test) of more than 300, in particular more than 350 and preferably more than 400 MPa.

17. Starting glass which comprises the components of the glass ceramic according to any one of claims 2 to 10 and comprises nuclei for the crystallization of scheelite in the form of Ca scheelite (CaWO₄) and/or Sr scheelite (SrWO₄) and/or for the crystallization of powellite in the form of Ca powellite (CaMoO₄) and/or Sr powellite (SrMoO₄).

18. Process for producing the glass ceramic according to any one of claims 1 to 16, in which a starting glass comprising the components of the glass ceramic, in particular the starting glass with nuclei according to claim 17, is subjected to at least one heat treatment in the range from 550 to 940°C.

19. Process according to claim 18, in which
(a) the starting glass is subjected to a heat treatment at a temperature of from 460 to 530°C for a duration of from 10 to 30 min in order to form the starting glass with nuclei, and
(b) the starting glass with nuclei is subjected to at least one heat treatment at a temperature of from 580 to 920°C for a duration of from 10 to 60 min in order to form the lithium silicate glass ceramic.

20. Use of the glass ceramic according to any one of claims 1 to 16 or the starting glass with nuclei according to claim 17 as dental material, in particular for the production of dental restorations.

## Revendications

1. Vitrocéramique à base de silicate de lithium, qui contient 0 à 6,0 % en poids de CaO et contient du silicate de lithium en tant que phase cristalline principale et de la scheelite sous forme de scheelite de Ca (CaWO₄) et/ou scheelite de Sr (SrWO₄) et/ou de la powellite sous forme de powellite de Ca (CaMoO₄) et/ou powellite de Sr (SrMoO₄) en tant qu'autres phases cristallines.

2. Vitrocéramique selon la revendication 1, qui contient 8,0 à 20,0, en particulier 11,0 à 17,0 et de préférence 13,0 à 15,0 % en poids de Li₂O.

3. Vitrocéramique selon la revendication 1 ou 2, qui contient 51,0 à 77,0, en particulier 55,0 à 75,0 et de préférence 64,0 à 74,0 % en poids de SiO₂.

4. Vitrocéramique selon l'une quelconque des revendications 1 à 3, dans laquelle la quantité combinée de CaO et SrO vaut en particulier 0,1 à 10,0, de préférence 0,5 à 7,0 et de façon particulièrement préférée 1,0 à 5,0 % en poids.

5. Vitrocéramique selon l'une quelconque des revendications 1 à 4, qui contient 0,1 à 4,0 et de préférence 0,5 à 2,0 % en poids de CaO.

6. Vitrocéramique selon l'une quelconque des revendications 1 à 5, qui contient 0 à 10,0, en particulier 0,1 à 7,0 et de préférence 0,5 à 4,0 % en poids de SrO.

7. Vitrocéramique selon l'une quelconque des revendications 1 à 6, qui contient 0 à 12,0, en particulier 1,0 à 8,0 et de préférence 3,0 à 5,0 % en poids de MoO₃.

8. Vitrocéramique selon l'une quelconque des revendications 1 à 7, qui contient 0 à 22,0, en particulier 1,0 à 14,0 et de préférence 4,0 à 7,0 % en poids de WO₃.

9. Vitrocéramique selon l'une quelconque des revendications 1 à 8, qui contient 1,5 à 6,0, en particulier 2,0 à 5,0 et de préférence 2,5 à 4,5 % en poids de P₂O₅.

10. Vitrocéramique selon l'une quelconque des revendications 1 à 9, qui contient au moins un et de préférence la totalité des composants suivants en les quantités indiquées :
| Composant | % en poids |
|---|---|
| SiO₂ | 51,0 - 77,0 |
| Li₂O | 8,0 - 20,0 |
| SrO | 0 - 10,0 |
| MoO₃ | 0 - 12,0 |
| WO₃ | 0 - 22,0 |
| Me^{I}₂O | 0 - 6,0 |
| Me^{II}O | 0 - 4,0 |
| M^{III}₂O₃ | 0 - 11,0 |
| Me^{IV}O₂ | 0 - 11,0 |
| P₂O₅ | 1,5 - 6,0 |
| Me^{V}₂O₅ | 0 - 11,5 |
| F | 0 - 2,0, |
Me^{I}₂O désignant un oxyde de métal alcalin à l'exception de Li₁O,
Me^{II}O désignant un oxyde d'élément divalent à l'exception de CaO et SrO,
Me^{III}₂O₃ désignant un oxyde d'élément trivalent,
Me^{IV}O₂ désignant un oxyde d'élément tétravalent à l'exception de SiO₂ et
Me^{V}₂O₅ désignant un oxyde d'élément pentavalent à l'exception de P₂O₅.

11. Vitrocéramique selon l'une quelconque des revendications 1 à 10, qui contient du silicate de lithium sous forme de disilicate de lithium et/ou métasilicate de lithium.

12. Vitrocéramique selon l'une quelconque des revendications 1 à 11, qui contient de la scheelite sous forme de scheelite de Ca (CaWO₄) et/ou scheelite de Sr (SrWO₄).

13. Vitrocéramique selon l'une quelconque des revendications 1 à 12, qui contient de la powellite sous forme de powellite de Ca (CaMoO₄) et/ou powellite de Sr (SrMoO₄).

14. Vitrocéramique selon l'une quelconque des revendications 1 à 13, qui entre en fluorescence sous excitation par de la lumière de longueur d'onde 254 nm.

15. Vitrocéramique selon l'une quelconque des revendications 1 à 14, qui présente une opacité aux rayons X selon EN ISO 4049 de plus de 120 %, en particulier plus de 150 % et de préférence plus de 180 %.

16. Vitrocéramique selon l'une quelconque des revendications 1 à 15, qui présente une résistance à la rupture biaxiale selon ISO 6872 (2015) (essai au piston sur trois billes) de plus de 300, en particulier plus de 350 et de préférence plus de 400 MPa.

17. Verre de départ, qui contient les composants de la vitrocéramique selon l'une quelconque des revendications 2 à 10 et contient des germes pour la cristallisation de scheelite sous forme de scheelite de Ca (CaWO₄) et/ou scheelite de Sr (SrWO₄) et/ou pour la cristallisation de powellite sous forme de powellite de Ca (CaMoO₄) et/ou powellite de Sr (SrMoO₄).

18. Procédé pour la production de la vitrocéramique selon l'une quelconque des revendications 1 à 16, dans lequel on soumet un verre de départ, qui contient les composants de la vitrocéramique, en particulier le verre de départ comportant des germes selon la revendication 17, à au moins un traitement thermique dans la plage de 550 à 940 °C.

19. Procédé selon la revendication 18, dans lequel
(a) on soumet le verre de départ à un traitement thermique à une température de 460 à 530 °C pendant une durée de 10 à 30 min, afin de former le verre de départ comportant des germes, et
(b) on soumet le verre de départ comportant des germes à au moins un traitement thermique à une température de 580 à 920 °C pendant une durée de 10 à 60 min, afin de former la vitrocéramique à base de silicate de lithium.

20. Utilisation de la vitrocéramique selon l'une quelconque des revendications 1 à 16 ou du verre de départ comportant des germes selon la revendication 17 en tant que matériau dentaire, en particulier pour la fabrication de restaurations dentaires.
